# EUROPEAN PATENT SPECIFICATION

(11) **EP 4 284 472 B1**
(45) Date of publication and mention of the grant of the patent: **11.09.2024**
(21) Application number: 21844052.7
(22) Date of filing: 20.12.2021
(51) Int. Cl.: A61M 11/06, A61M 11/00, A61H 35/04, A61M 15/00, A61M 3/02, B05B 7/24, B05B 7/00

(54) **CAPSULE AND DELIVERY ASSEMBLY FOR THE DELIVERY OF A NEBULIZED SUBSTANCE**
KAPSEL UND ABGABEANORDNUNG ZUR ABGABE EINER ZERSTÄUBTEN SUBSTANZ
CAPSULE ET ENSEMBLE D'ADMINISTRATION POUR L'ADMINISTRATION D'UNE SUBSTANCE NÉBULISÉE

(30) Priority: 29.01.2021 IT 202100001916
(43) Date of publication of application: 06.12.2023
(73) Proprietor: 3A Health Care S.R.L., 25017 Lonato del Garda, BRESCIA (IT)
(72) Inventor: BINAGHI, Marco, 25017 Lonato del Garda, Brescia (IT); PELLIZZON, Alice, 25017 Lonato del Garda, Brescia (IT); SECCO, Guido, 25017 Lonato del Garda, Brescia (IT); GUARAGNA, Giacomo, 25017 Lonato del Garda, Brescia (IT); MONEGHINI, Graziano, 25017 Lonato del Garda, Brescia (IT); ABATE, Simone, 25017 Lonato del Garda, Brescia (IT); FRACCAROLI, Davide, 25017 Lonato del Garda, Brescia (IT)
(74) Representative: Chimini, Francesco
(86) International application number: PCT/IB2021/062007
(87) International publication number: WO 2022/162458

(56) References cited:
- WO-A2-2013/030117
- WO-A2-2013/072856
- CN-U- 208 877 247
- GB-A- 2 531 789
- IT-A1- 201800 009 586

## Description

This invention relates to the field of devices for nebulizing a substance to perform nasal irrigation and aerosol therapy treatments.

In some embodiments, these devices comprise a base body provided with a nozzle suitable to be fluidly connected to a source of compressed air. The base body defines a chamber suitable to accommodate the substance to be nebulized. The substance can be introduced into the chamber by taking it from a container, such as a vial, or it can be contained in a disposable capsule suitable to be inserted and punctured inside the chamber.

A lid closes the chamber and is equipped with a mouth for dispensing the nebulized substance.

In order to nebulize the substance in a liquid state which is contained in the chamber, a nozzle cover, commonly called a 'pisper,' is fitted onto the nozzle, which forms with the nozzle at least one suction channel for the substance, which is sucked towards the constriction at the end of the nozzle cover, exploiting the Venturi effect produced by the pressurized air passing through the nozzle.

For aerosol therapy applications, the nozzle cover is usually equipped with a nebulizer element which extends in the form of a pin just above the end constriction and which, by mechanical impact, breaks up particles of the substance exiting said constriction, causing the substance to be nebulized. Examples of capsule are disclosed in the following prior art documents IT 2018 0000 9586 A1, WO 2013/030117 A2, GB 2 531 789 A, CN 208 877 247 U and WO 2013/072856 A2. The document IT 2018 0000 9586 A1, for example, discloses a single-use capsule for the generation of an aerosol. The capsule comprises: an external envelope having at least one inlet for a compressed gas and one outlet for the aerosol; a spray volume defined inside the external envelope and communicating with said inlet and with said outlet; a containment volume defined inside the envelope external and containing a liquid to be nebulized; an aerosol generator contained within the nebulization volume and designed to nebulize the liquid to be nebulized with the compressed gas to generate the aerosol; a first openable film applied to the outer casing to seal the entrance; and a second openable film applied to the outer casing to seal the outlet. A device for performing an inhalation therapy is also disclosed. The device comprises: the above capsule; a generator apparatus for generating a compressed gas; a connecting element adapted to connect an outlet of the generator apparatus to the inlet of the capsule; and an aerosol dispenser connected to the outlet of the capsule.

An object of this invention is to propose an assembly for nasal irrigation and aerosol therapy to make cleaning and sanitizing of the delivery device easier, faster, and safer.

Another object of the invention is to provide an assembly that allows for effective recovery of the nebulized substance that does not reach the delivery mouth, thereby ensuring the maximum performance of the device.

These objects are achieved with a capsule according to claim 1 and a dispensing assembly according to claim 10.

The features and advantages of this invention will be more readily understandable from the following description of its preferred and non-limiting examples of embodiments, wherein:
- Fig. 1 is an exploded perspective view of a dispensing assembly according to an embodiment of this invention, suitable for nasal irrigation applications;
- Fig. 2 is an axial section of the delivery assembly of Fig. 1;
- Fig. 3 is a perspective view of only the capsule of the assembly of the preceding figures;
- Fig. 4 is an exploded perspective view of a dispensing assembly according to another embodiment of this invention, for example suitable for aerosol therapy applications;
- Fig. 5 is an axial section of the delivery assembly from Fig. 4; and
- Fig. 6 is a perspective view of only the capsule of the assembly of the preceding figures;

In the following description and in the attached drawings, the elements or parts of elements in common among various embodiments of the invention will be indicated with the same numerical references.

Further, the terms "upper" and "lower" are used in reference to the delivery device being at rest and positioned on a support plane.

With reference to the aforesaid figures, 1; 1001 is used to globally refer to a delivery assembly of a nebulized substance, for example a substance for nasal irrigation or for aerosol therapy.

The assembly 1; 1001 comprises a capsule 10; 1010 containing the substance in a liquid state and a delivery device 4; 400, also known as an "ampoule," cooperating with the capsule 10; 1010 to nebulize the substance contained therein.

According to an aspect of the invention, when the capsule 10; 1010 is associated with the delivery device 4; 400, the capsule 10; 1010 in turn forms a part of the delivery device 4; 400. In other words, as will be described in more detail below, the capsule 10; 1010 is not inserted into a respective chamber formed in the dispensing device, as in some delivery devices according to the state of the art, but forms a structural element connecting two separate parts of the delivery device 4; 400.

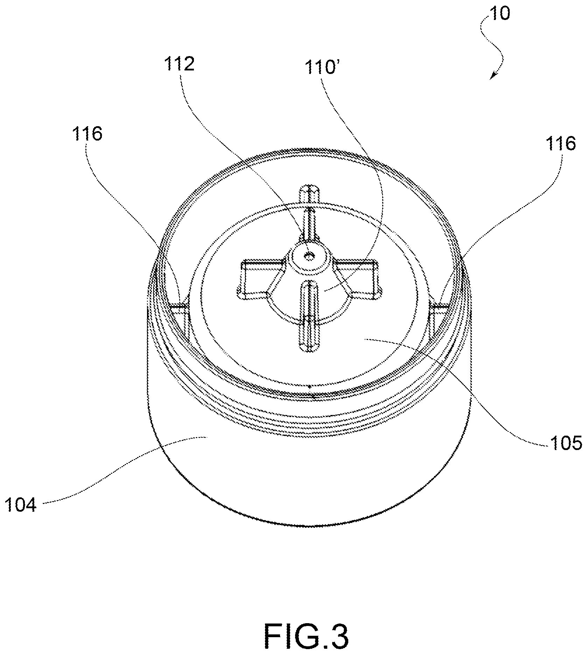

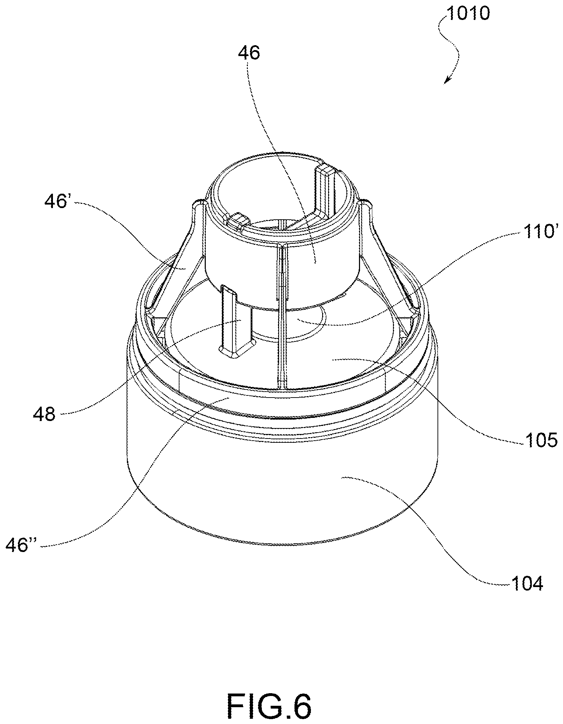

With particular reference to Fig. 3 and 6, the structure of the capsule 10; 1010 will thus first be described.

The capsule 10; 1010 comprises an inner capsule body 102, e.g., cylindrical in shape, and an outer side wall 104 surrounding the inner capsule body 102.

In an embodiment, the outer side wall 104 is arranged coaxially with the inner capsule body 102.

The inner capsule body 102 forms at least one closed chamber 106 suitable to contain a respective substance to be nebulized. In some embodiments, multiple closed chambers 106 can be formed in the inner body 102, for example in the form of hermetically separated sectors, each containing a respective substance.

The closed chamber 106 (or the set of closed chambers) is delimited at the bottom by a capsule bottom wall 108.

At least one pierceable and/or tearable and/or peelable portion is obtained in the capsule bottom wall 108 to allow for the outflow of the substance from the capsule. The substance exiting the at least one enclosed chamber 106 will be collected in a substance catch basin of the delivery device, as will be further described below.

A capsule nozzle 110 ending with a substance outlet hole 112 at the top is formed in the inner capsule body 102.

The capsule nozzle 110 is suitable to be fitted on a device nozzle 12 of the delivery device 4; 400, described further hereinafter, so as to define with the device nozzle 12 at least one substance suction channel 122 for suction of the substance towards the outlet hole 112 by means of a Venturi effect.

The closed chamber 106 is formed around the capsule nozzle 110. The outer wall 104 delimits, with the inner capsule body 102, a substance recovery channel 114.

In an embodiment, the outer wall 104 is a cylindrical wall that delimits, with the inner capsule body 102, an annular gap forming the substance recovery channel 114.

For example, the outer wall 104 is connected to the inner capsule body 102 by means of septa or radial arms 116.

The outer wall 104 forms a lower edge 104a suitable to connect to a lower portion of the delivery device 4; 400.

Further, the outer wall 104 forms an upper edge 104b suitable to connect with an upper portion of the delivery device 4; 400.

In an embodiment, the capsule body 102 and the capsule nozzle 110 are made in one piece in a single body, for example of a plastic material.

In other embodiments, the capsule nozzle 110 and the capsule 102 could be made separately and subsequently be connected to each other, such as by shape and/or force coupling or other attachment systems, such as screw, bayonet, etc.

Because the capsule 10; 1010 is configured to overlap and surround the device nozzle 12, the capsule nozzle 110 penetrates the entire capsule body 102 and protrudes, with a conical top portion 110' thereof, from a top wall 105 of the capsule body.

In an embodiment, the capsule body 102 thus has a substantially toroidal shape.

Obviously, the capsule body 102 could also have other shapes than the circular annular shape depicted in the drawings, for example it can be formed according to the shape of the body of the delivery device with which the capsule is associated.

As mentioned above, in some embodiments, the closed capsule chamber 106 can be divided into two or more compartments to contain respective different substances, for example different drugs, which can for example be mixed upon opening.

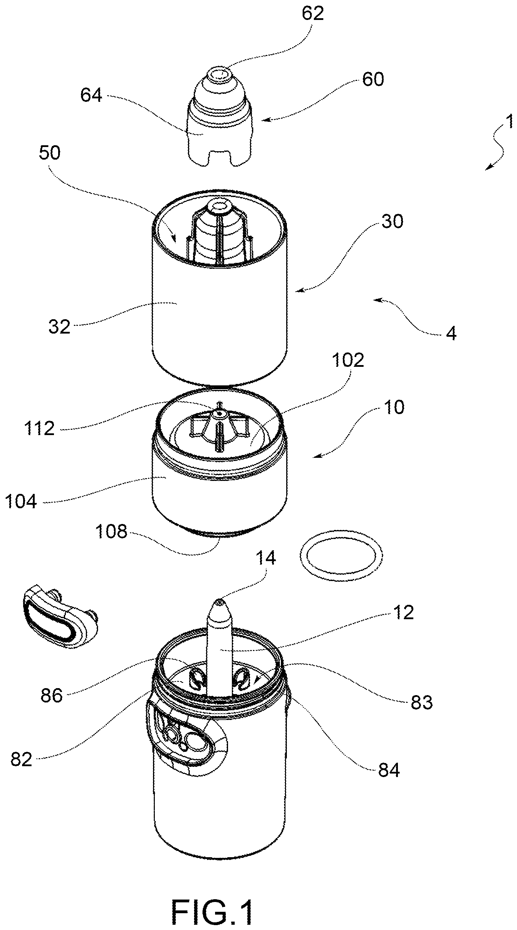

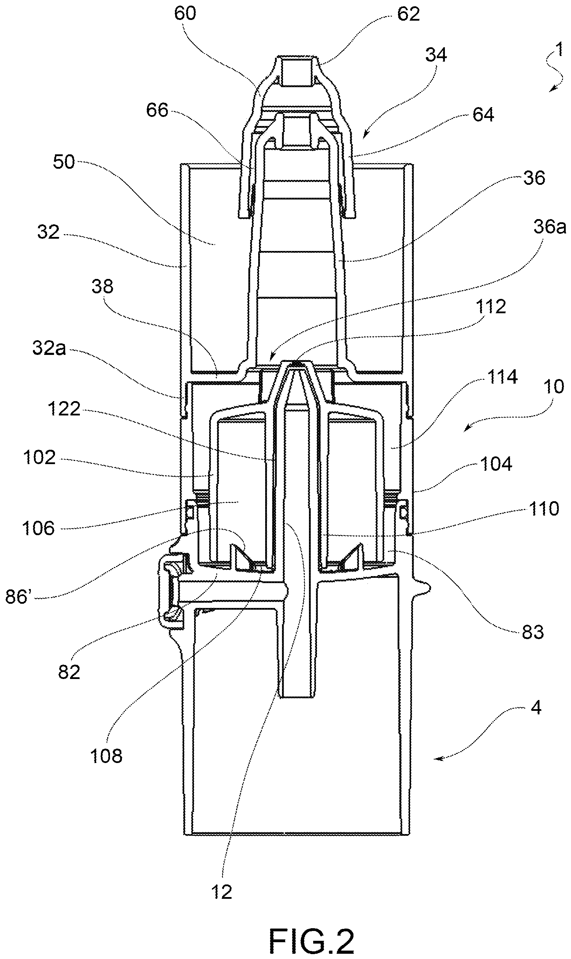

In the embodiment shown in Fig. 1-3, the capsule 10 is configured to make the delivery device 4 suitable to perform a nasal irrigation. In this case, the substance exiting the outlet hole 112 is in the form of particles sized for this type of application.

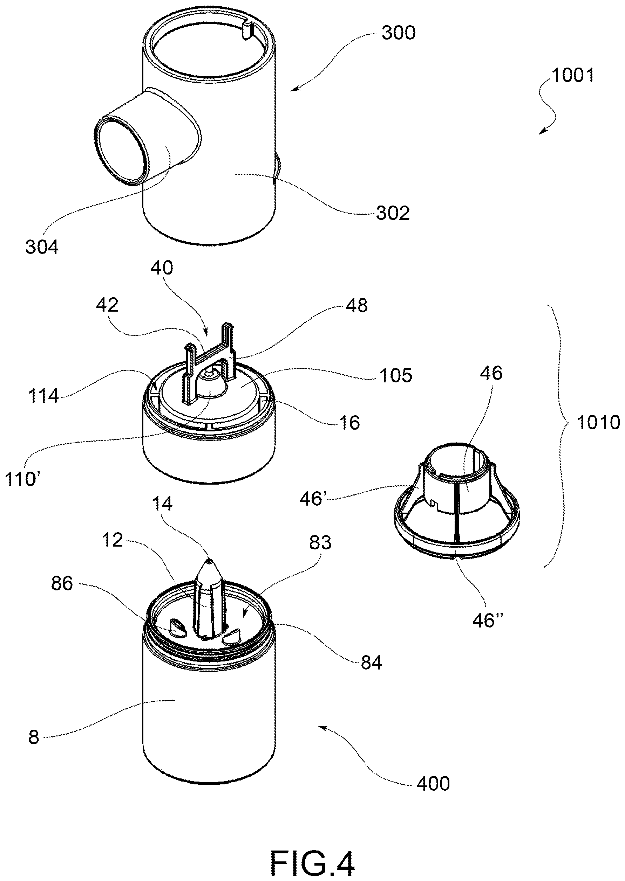

In an embodiment illustrated in Fig. 4-6, the capsule 1010 is configured to make the delivery device 400 suitable for aerosol therapy applications. In this case, it is necessary to further reduce the particle size of the substance by means of special nebulization means 40.

In particular, the capsule body 102 supports nebulization means 40 suitable to cause a nebulization of the substance exiting from the capsule nozzle 110 via the substance's impact with these nebulization means 40.

In an embodiment, the nebulization means 40 comprise a transverse portion 42 extending orthogonally relative to a nozzle axis X parallel to the outlet direction of the substance from the capsule nozzle 110, and a breaking pin 44 extending from the transverse portion 42, coaxially to the nozzle axis, so as to break up the particles of the substance exiting the capsule nozzle 110.

In an embodiment, the capsule body 102 further supports a cylindrical element 46 that extends coaxially around the nebulization means 40. The height of this cylindrical element 46 determines, in conjunction with the delivery device, the size of the particulate matter that is conveyed to the delivery mouth of the delivery device.

In an embodiment, the cylindrical element 46 is integral with the nebulization means 40.

In an embodiment, the nebulization means 40 are connected to the capsule body by at least two parallel support arms 48 extending from the top wall 105 of the capsule body 102, parallel to the capsule nozzle 110.

In an embodiment, the cylindrical element 46 is connected, by means of radial arms 46', to a support ring 46" that engages the top edge 104b of the outer side wall 104 with shape and/or force coupling.

In an embodiment, the pierceable and/or tearable and/or peelable portion 108 is made of a film or with a region having a thickness less than the thickness of the capsule bottom wall 108.

Returning now to the assembly 1; 1001, in a general embodiment, the delivery device 4; 400 comprises a base body 8 provided with a device nozzle 12 suitable to be fluidly connected to a source of compressed air.

The device nozzle 12 ends in a compressed air delivery hole 14 and is configured to be inserted into the capsule nozzle 110.

The base body 8 forms a concave wall 82 for collecting substance. From this concave wall 82 the device nozzle 12 extends.

The concave wall 82 is peripherally delimited by an upper capsule coupling edge 84 suitable to obtain a shape and/or force coupling with the lower edge 104a of the outer side wall 104.

The concave wall 82 forms, with the upper capsule coupling edge 84, a substance catch basin 83.

Further, the concave wall 82 is provided with chamber opening means 86 suitable to obtain at least one opening in the pierceable and/or tearable and/or peelable portion by a related movement between the capsule 10 and the base body 8.

The dispensing device 4 further comprises an upper dispensing body 30; 300 having a side wall 32; 302 forming a lower capsule connecting edge 32a; 302a suitable to obtain a shape and/or force coupling with the upper edge 104b of the outer side wall of the capsule.

Thus, the capsule 10; 1010 serves as a connecting element between the base body 8 and the upper delivery body 30; 300.

In an embodiment, the relative movement between the capsule 10; 1010 and the base body 8 is achieved by axial translation or roto-translation of the capsule 10 with respect to the base body 8. For example, said relative movement coincides with or is a part of the movement that allows the capsule 10 to be connected to the base body 8.

The capsule opening means 86 are suitable to obtain one or more passages in the capsule bottom wall 108 for the outflow of the substance contained in the closed chamber 106 into the substance catch basin 83.

In order to make one or more passages in the capsule bottom wall, the opening means can pierce or tear or peel one or more portions of the capsule end wall.

In a variant embodiment, the opening means 86 can also cause the capsule bottom wall to separate or detach from the capsule body. Such separation or detachment can be due to a compression or a traction exerted on the capsule bottom wall.

In an embodiment, the capsule opening means 86 comprise one or more piercing tips.

In an embodiment, a passage 86' suitable to drain the substance from the capsule into the catch basin 83 is formed in each piercing tip.

In a variant embodiment, the capsule opening means 86, for example in the form of piercing tips, can be formed in a yielding bottom of the capsule obtained below the capsule bottom wall 108.

Note that the concave bottom wall 82 and/or the capsule bottom wall 108 are shaped so as to form the substance catch basin 83 between the concave bottom wall 82 and the capsule bottom wall 108, even when the capsule 10; 1010 is, for example, pressed flat against the concave bottom wall 82.

The upper delivery body 30; 300 is provided with a delivery mouth 34; 304 of the nebulized substance suitable to convey the nebulized substance towards the outside of the capsule.

In an embodiment illustrated in Fig. 1-2, the delivery device 4 is configured to perform a nasal irrigation. The upper delivery body 30 forms a bottom wall 38, an inner siphon 36 which extends from the bottom wall 38 and which has an inlet mouth 36a arranged to receive the substance exiting from the capsule nozzle 110.

The internal siphon 36 ends with an outlet mouth 36b, opposite the inlet mouth. In an assembly configuration of the assembly, the side wall 32, the bottom wall 38 and inner siphon 36 delimit a mucus collection chamber 50 fluidly separated from the underlying capsule.

The bottom wall 38 and the inlet mouth 36a of the inner siphon 36 are spaced apart from the capsule 10 to allow a return of the nebulized substance from the inner siphon 36 to the concave substance collection wall 82 through the substance recovery channel 114.

In other words, the nebulized substance that exits from the outlet hole 112 and does not reach the outlet mouth 36b can fall back into the catch basin formed by the concave wall 82 and then be recovered and again sucked up by the Venturi effect.

In an embodiment, a delivery cap 60 is mounted on the inner siphon 36 that ends in a delivery tip 62 suitable for insertion into a nostril of the user. The delivery cap 60 has a side wall 64 located around a portion of the top of the inner siphon 36. Between this side wall 64 and the top portion, drainage channels 66 are obtained to drain the mucus into the mucus collection chamber 50.

Accordingly, the liquid flowing down the nostril of the user can drain onto the outer wall of the cap 60 or, if it enters the delivery tip 62, is diverted into the drainage channels 66 by the flow of compressed air directed towards the delivery tip 62.

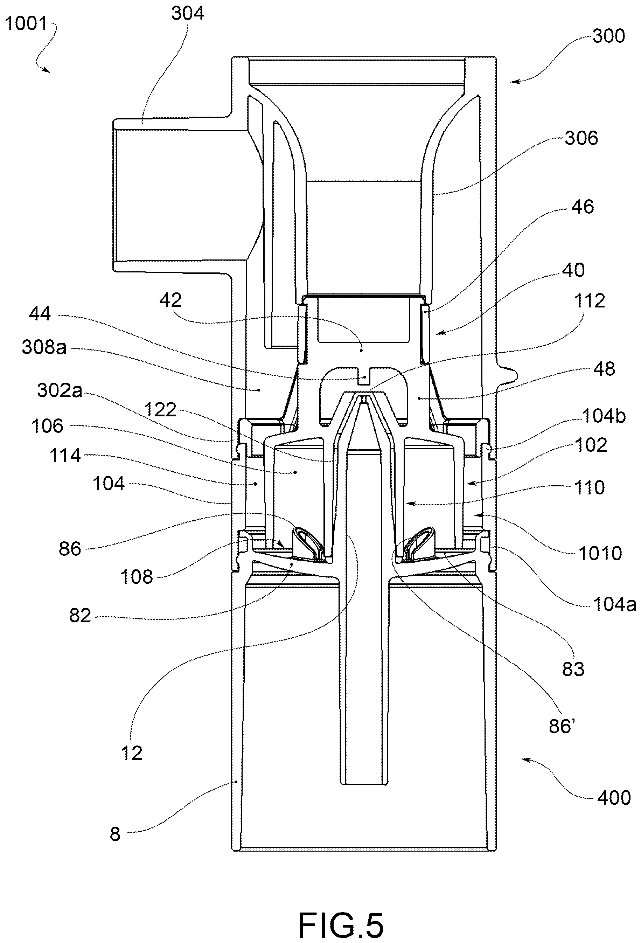

Fig. 4 and 5 show a delivery device 400 configured to perform aerosol therapy treatments. In this case, the capsule 1010 is provided with the nebulization means 40.

The delivery device 400 has the upper delivery body 300 equipped with a tubular portion 306 that is open above to the outside and below to the nebulization means 40 (when the upper delivery body 300 is connected to the capsule). This tubular portion 306 allows for an intake of air from the outside by the Venturi effect. The upper delivery body 300 forms a delivery mouth 304 in communication with a nebulization chamber 308 formed around the tubular portion 306.

Also in this case, in an assembly configuration of the assembly, the nebulization chamber 308 is in fluid communication with the substance recovery channel 114 of the capsule 1010. Therefore, the nebulized substance that does not reach the delivery mouth 304 falls back into the catch basin formed by the concave wall 82 to be sucked in and nebulized again.

It is evident that the delivery assembly and the capsule described above enable the intended objects to be achieved.

In particular, the delivery device 4; 400 has a simplified structure and is easier to clean and sanitize after use.

Due to the special structure of the capsule, which allows an effective recovery of the nebulized substance through the recovery channel 114, all the substance contained therein is nebulized.

The assembly is assembled quickly and easily: the user only has to couple the capsule to the base body and the upper body to the capsule.

To the embodiments of the capsule and the delivery assembly according to the invention a person skilled in the art, in order to meet contingent needs, can make modifications, adaptations, and substitutions of elements with others that are functionally equivalent, without going beyond the scope of the following claims.

## Claims

1. A capsule (10, 1010) containing at least one liquid substance for aerosol therapy or for nasal irrigation, comprising an inner capsule body (102) and an outer side wall (104) surrounding the inner capsule body (102), wherein the inner capsule body forms at least one closed chamber (106) containing the at least one substance and delimited below by a capsule bottom wall (108) in which at least one pierceable and/or tearable and/or peelable portion is obtained to allow the outflow of the substance from the capsule, wherein in the inner capsule body there is a capsule nozzle (110) ending above with an outlet hole (112) of the substance from the capsule, the capsule nozzle (110) being suitable to be fitted onto a device nozzle (12) of a delivery device (4; 400) so as to define with the device nozzle (12) at least one substance suction channel (122) for a suction of the substance towards the outlet hole (112) by Venturi effect, the closed chamber (106) being obtained around the capsule nozzle (110), wherein the outer wall (104) defines, with the inner capsule body (102), a substance recovery channel (114), and wherein the outer wall (104) forms a lower edge (104a) suitable to connect to a lower portion of the delivery device and an upper edge (104b) suitable to connect to an upper portion of the delivery device.

2. A capsule according to claim 1, wherein the capsule body (102) supports nebulization means (40) suitable to cause a nebulization of the substance exiting from the capsule nozzle by means of the impact of the substance with said nebulization means (40).

3. A capsule according to claim 2, wherein said nebulization means (40) comprise a transverse portion (42) extending orthogonally with respect to a nozzle axis (X) parallel to the exit direction of the substance from the capsule nozzle, and a breaking pin (44) extending from said transverse portion (42), coaxially to the nozzle axis, so as to break the particles of the substance exiting from the capsule nozzle (110).

4. A capsule according to claim 2 or 3, wherein the capsule body (102) further supports a cylindrical element (46) extending coaxially around the nebulization means (40) .

5. A capsule according to any one of claims 2-4, wherein the nebulization means (40) are connected to the capsule body through at least two parallel support arms (48) extending from a top wall (105) of the capsule body, parallel to the capsule nozzle.

6. A capsule according to claim 4 or claim 5 when dependent on claim 4, wherein the cylindrical element (46) is connected, through radial arms (46'), to a support ring (46") which engages by shape and/or force coupling with the upper edge (104b) of the outer side wall (104).

7. A capsule according to any one of the preceding claims, wherein the capsule nozzle (110) axially crosses the capsule body (102) so as to give the capsule body (102) a toroidal shape.

8. A capsule according to any one of the preceding claims, wherein said pierceable and/or tearable and/or peelable portion is made with a film or with a region having a thickness smaller with respect to the thickness of the capsule bottom wall.

9. A capsule according to any one of the preceding claims, wherein the outer wall (104) is connected to the inner capsule body (102) through two or more radial septa (116) .

10. A delivery assembly for a nebulized substance for aerosol therapy or for nasal irrigation, comprising a capsule (10; 1010) according to any one of the preceding claims, containing the substance in the liquid state, and a delivery device (4; 400) comprising:
- a base body (8) provided with a device nozzle (12) suitable to be fluidly connected to a compressed air source, the device nozzle (12) having a delivery hole (14) of the compressed air and being configured to fit into the capsule nozzle (110), the base body (8) defining a concave bottom wall (82) for collecting the substance from which the device nozzle (12) extends, the concave bottom wall (82) being peripherally delimited by an upper capsule coupling edge (84) suitable to obtain a shape and/or force coupling with the lower edge (104a) of the outer side wall (104) of the capsule and being provided with chamber opening means (86) suitable to obtain at least one opening in the pierceable and/or tearable and/or peelable portion due to a relative movement between the capsule and the base body;
- an upper delivery body (30; 300) having a side wall (32; 302) forming a lower edge (32a; 302a) for connecting the capsule suitable to obtain a shape and/or force coupling with the upper edge (104b) of the outer side wall of the capsule, the upper delivery body being provided with a delivery mouth (34; 304) of the nebulized substance suitable to convey to the outside the nebulized substance exiting from the capsule.

11. Assembly according to the preceding claim, wherein the upper delivery body (30) forms a bottom wall (38), an inner siphon (36) extending from said bottom wall (38) and which has an inlet mouth (36a) arranged so as to receive the substance exiting from the capsule nozzle (112) and an outlet mouth (36b) opposite the inlet mouth, and wherein, in an assembly configuration of the assembly, the side wall (32), the bottom wall (38) and the inner siphon (36) delimit a mucus collection chamber (50) fluidly separated from the underlying capsule, the bottom wall (38) and the inlet mouth (36a) of the inner siphon (36) being spaced apart from the capsule (10) to allow a return of the nebulized substance from the inner siphon (36) to the concave wall (82) for collecting the substance through the substance recovery channel (114).

12. Assembly according to the preceding claim, wherein a delivery cap (60) is mounted on the inner siphon (36), the cap ending with a delivery tip (62) suitable to be inserted in a nostril of the user, the delivery cap (60) having a side wall (64) placed around a top portion of the inner siphon (36), mucus drainage channels (66) being obtained between said side wall and said top portion in the mucus collection chamber (50).

13. Assembly according to claim 10, wherein the capsule (1010) is provided with nebulization means (40) and the upper delivery body (300) is provided with a tubular portion (306) open above towards the outside and below towards the nebulization means (40), when the upper delivery body (300) is connected to the capsule, so as to allow a suction of air from the outside by Venturi effect, the delivery mouth (304) being in communication with a nebulization chamber (308) obtained around the tubular portion (306).

14. Assembly according to the preceding claim, wherein, in an assembly configuration of the assembly, the nebulization chamber (308) is in fluid communication with the substance recovery channel (114) of the capsule (10).

## Patentansprüche

1. Kapsel (10, 1010), die zumindest eine flüssige Substanz zur Aerosoltherapie oder zur Nasenspülung enthält, umfassend einen inneren Kapselkörper (102) und eine Außenseitenwand (104), die den inneren Kapselkörper (102) umgibt, wobei der innere Kapselkörper zumindest eine geschlossene Kammer (106) bildet, die die zumindest eine Substanz enthält und unten durch eine Kapselbodenwand (108) begrenzt ist, in der zumindest ein durchstechbarer und/oder abreißbarer und/oder abziehbarer Abschnitt vorhanden ist, um das Ausfließen der Substanz aus der Kapsel zu ermöglichen, wobei sich in dem inneren Kapselkörper eine Kapseldüse (110) befindet, die oben mit einem Auslassloch (112) der bzw. für die Substanz aus der Kapsel endet, wobei die Kapseldüse (110) geeignet ist, auf eine Vorrichtungsdüse (12) einer Abgabevorrichtung (4; 400) aufgepasst zu werden, um mit der Vorrichtungsdüse (12) zumindest einen Substanzansaugkanal (122) für ein Ansaugen der Substanz zu dem Auslassloch (112) hin durch Venturi-Effekt zu definieren, wobei die geschlossene Kammer (106) um die Kapseldüse (110) herum vorhanden ist, wobei die Außenwand (104) mit dem inneren Kapselkörper (102) einen Substanzrückführungskanal (114) definiert und wobei die Außenwand (104) eine untere Kante bzw. einen unteren Rand (104a), die bzw. der sich zum Verbinden mit einem unteren Abschnitt der Abgabevorrichtung eignet, und eine obere Kante bzw. einen oberen Rand (104b) bildet, die bzw. der sich zum Verbinden mit einem oberen Abschnitt der Abgabevorrichtung eignet.

2. Kapsel nach Anspruch 1, wobei der Kapselkörper (102) Zerstäubungsmittel (40) stützt bzw. trägt, die geeignet sind, eine Zerstäubung der aus der Kapseldüse austretenden Substanz mittels bzw. durch Aufprall der Substanz auf die Zerstäubungsmittel (40) zu bewirken.

3. Kapsel nach Anspruch 2, wobei die Zerstäubungsmittel (40) einen Querabschnitt (42), der sich orthogonal in Bezug auf eine Düsenachse (X) parallel zu der Austrittsrichtung der Substanz aus der Kapseldüse erstreckt, und einen Brechstift (44) umfassen, der sich von dem Querabschnitt (42) koaxial zu der Düsenachse erstreckt, um die Partikel der Substanz zu brechen, die aus der Kapseldüse (110) austreten.

4. Kapsel nach Anspruch 2 oder 3, wobei der Kapselkörper (102) ferner ein zylindrisches Element (46) stützt bzw. trägt, das sich koaxial um die Zerstäubungsmittel (40) erstreckt.

5. Kapsel nach einem der Ansprüche 2-4, wobei die Zerstäubungsmittel (40) mit dem Kapselkörper durch zumindest zwei parallele Stütz- bzw. Trägerarme (48) verbunden sind, die sich von einer oberen Wand (105) des Kapselkörpers parallel zu der Kapseldüse erstrecken.

6. Kapsel nach Anspruch 4 oder Anspruch 5, wenn von Anspruch 4 abhängig, wobei das zylindrische Element (46) durch radiale Arme (46') mit einem Stütz- bzw. Trägerring (46") verbunden ist, der durch Form- und/oder Kraftkopplung mit der oberen Kante (104b) der Außenseitenwand (104) in Eingriff ist.

7. Kapsel nach einem der vorhergehenden Ansprüche, wobei die Kapseldüse (110) den Kapselkörper (102) axial kreuzt, um dem Kapselkörper (102) eine toroidale Form zu verleihen.

8. Kapsel nach einem der vorhergehenden Ansprüche, wobei der durchstechbare und/oder abreißbare und/oder abziehbare Abschnitt mit bzw. aus einem Film bzw. einer Folie oder einem Bereich mit einer Dicke hergestellt ist, die in Bezug auf die Dicke der Kapselbodenwand geringer ist.

9. Kapsel nach einem der vorhergehenden Ansprüche, wobei die Außenwand (104) mit dem inneren Kapselkörper (102) durch zwei oder mehr radiale Septen (116) verbunden ist.

10. Abgabeanordnung für eine zerstäubte Substanz zur Aerosoltherapie oder zur Nasenspülung, umfassend eine Kapsel (10; 1010) nach einem der vorhergehenden Ansprüche, die die Substanz im flüssigen Zustand enthält, und eine Abgabevorrichtung (4; 400), umfassend:
- einen Basiskörper (8), der mit einer Vorrichtungsdüse (12) versehen ist, die geeignet ist, fluidisch mit einer Druckluftquelle verbunden zu werden, wobei die Vorrichtungsdüse (12) ein Abgabeloch (14) der bzw. für die Druckluft aufweist und konfiguriert ist, in die Kapseldüse (110) zu passen, wobei der Basiskörper (8) eine konkave Bodenwand (82) zum Auffangen der Substanz definiert, von der sich die Vorrichtungsdüse (12) erstreckt, wobei die konkave Bodenwand (82) peripher durch eine obere Kapselkopplungskante bzw. einen oberen Kapselkopplungsrand (84) begrenzt ist, die bzw. der geeignet ist, eine Form- und/oder Kraftkopplung mit der unteren Kante (104a) der Außenseitenwand (104) der Kapsel zu erhalten, und mit Kammeröffnungsmitteln (86) versehen ist, die geeignet sind, zumindest eine Öffnung in dem durchstechbaren und/oder abreißbaren und/oder abziehbaren Abschnitt aufgrund einer relativen Bewegung zwischen der Kapsel und dem Basiskörper zu erhalten;
- einen oberen Abgabekörper (30; 300) mit einer Seitenwand (32; 302), die eine untere Kante bzw. einen unteren Rand (32a; 302a) zum Verbinden der Kapsel bildet, die geeignet ist, eine Form- und/oder Kraftkopplung mit der oberen Kante (104b) der Außenseitenwand der Kapsel zu erhalten, wobei der obere Abgabekörper mit einer Abgabemündung (34; 304) für die zerstäubte Substanz versehen ist, die geeignet ist, die aus der Kapsel austretende zerstäubte Substanz nach außen zu befördern.

11. Anordnung nach dem vorhergehenden Anspruch, wobei der obere Abgabekörper (30) eine Bodenwand (38) bildet, wobei sich ein innerer Siphon (36) von der Bodenwand (38) erstreckt und eine Einlassmündung (36a), die so angeordnet ist, dass sie die aus der Kapseldüse (112) austretende Substanz aufnimmt, und eine Auslassmündung (36b) gegenüberliegend bzw. entgegengesetzt zu der Einlassmündung aufweist, und wobei in einer Anordnungskonfiguration der Anordnung die Seitenwand (32), die Bodenwand (38) und der innere Siphon (36) eine Schleimauffangkammer (50) begrenzen, die fluidisch von der darunterliegenden Kapsel getrennt ist, wobei die Bodenwand (38) und die Einlassmündung (36a) des inneren Siphons (36) von der Kapsel (10) beabstandet sind, um eine Rückführung der zerstäubten Substanz von dem inneren Siphon (36) zu der konkaven Wand (82) zum Auffangen der Substanz durch den Substanzrückführungskanal (114) zu ermöglichen.

12. Anordnung nach dem vorhergehenden Anspruch, wobei eine Abgabekappe (60) auf dem inneren Siphon (36) montiert ist, wobei die Kappe mit einer Abgabespitze (62) endet, die geeignet ist, in ein Nasenloch des Benutzers eingeführt zu werden, wobei die Abgabekappe (60) eine Seitenwand (64) aufweist, die um einen oberen Abschnitt des inneren Siphons (36) herum platziert ist, wobei zwischen der Seitenwand und dem oberen Abschnitt in der Schleimauffangkammer (50) Schleimabflusskanäle (66) vorhanden sind.

13. Anordnung nach Anspruch 10, wobei die Kapsel (1010) mit Zerstäubungsmitteln (40) versehen ist und der obere Abgabekörper (300) mit einem rohrförmigen Abschnitt (306) versehen ist, der oben nach außen hin und unten zu den Zerstäubungsmitteln (40) hin offen ist, und zwar wenn der obere Abgabekörper (300) mit der Kapsel verbunden ist, um ein Ansaugen von Luft von außen durch Venturi-Effekt zu ermöglichen, wobei die Abgabemündung (304) mit einer Zerstäubungskammer (308) in Kommunikation bzw. Verbindung ist, die um den rohrförmigen Abschnitt (306) herum vorhanden ist.

14. Anordnung nach dem vorhergehenden Anspruch, wobei in einer Anordnungskonfiguration der Anordnung die Zerstäubungskammer (308) in Fluidkommunikation bzw. -verbindung mit dem Substanzrückführungskanal (114) der Kapsel (10) ist.

## Revendications

1. Capsule (10, 1010) contenant au moins une substance liquide pour une aérosolthérapie ou pour une irrigation nasale, comprenant un corps interne (102) de capsule et une paroi latérale externe (104) entourant le corps interne (102) de capsule, dans laquelle le corps interne de capsule forme au moins une chambre close (106) contenant l'au moins une substance et délimitée au-dessous par une paroi de fond (108) de capsule dans laquelle au moins une partie perforable et/ou déchirable et/ou décollable est obtenue pour permettre la sortie de la substance hors de la capsule, dans laquelle dans le corps interne de capsule est présente une buse (110) de capsule se terminant au-dessus par un trou de sortie (112) de la substance hors de la capsule, la buse (110) de capsule étant appropriée pour être installée sur une buse (12) de dispositif d'un dispositif d'administration (4 ; 400) de manière à définir avec la buse (12) de dispositif au moins un canal d'aspiration de substance (122) pour permettre une aspiration de la substance vers le trou de sortie (112) par un effet Venturi, la chambre close (106) étant obtenue autour de la buse (110) de capsule, dans laquelle la paroi externe (104) définit, avec le corps interne (102) de capsule, un canal de récupération de substance (114), et dans laquelle la paroi externe (104) forme un bord inférieur (104a) approprié pour être raccordé à une partie inférieure du dispositif d'administration et un bord supérieur (104b) approprié pour être raccordé à une partie supérieure du dispositif d'administration.

2. Capsule selon la revendication 1, dans laquelle le corps (102) de capsule supporte des moyens de nébulisation (40) appropriés pour provoquer une nébulisation de la substance sortant de la buse de capsule au moyen du choc de la substance avec lesdits moyens de nébulisation (40).

3. Capsule selon la revendication 2, dans laquelle lesdits moyens de nébulisation (40) comprennent une partie transversale (42) s'étendant perpendiculairement par rapport à un axe de buse (X) parallèle à la direction de sortie de la substance depuis la buse de capsule, et une tige de rupture (44) s'étendant à partir de ladite partie transversale (42), coaxialement à l'axe de buse, de manière à briser les particules de la substance sortant de la buse (110) de capsule.

4. Capsule selon la revendication 2 ou 3, dans laquelle le corps (102) de capsule supporte en outre un élément cylindrique (46) s'étendant coaxialement autour des moyens de nébulisation (40).

5. Capsule selon l'une quelconque des revendications 2 à 4, dans laquelle les moyens de nébulisation (40) sont reliés au corps de capsule par l'intermédiaire d'au moins deux bras de support parallèles (48) s'étendant depuis une paroi de dessus (105) du corps de capsule, parallèle à la buse de capsule.

6. Capsule selon la revendication 4 ou la revendication 5 lorsqu'elle dépend de la revendication 4, dans laquelle l'élément cylindrique (46) est relié, par l'intermédiaire de bras radiaux (46'), à une bague de support (46") qui vient en prise par couplage de forme et/ou de force avec le bord supérieur (104b) de la paroi latérale externe (104).

7. Capsule selon l'une quelconque des revendications précédentes, dans laquelle la buse (110) de capsule croise axialement le corps (102) de capsule de manière à donner au corps (102) de capsule une forme toroïdale.

8. Capsule selon l'une quelconque des revendications précédentes, dans laquelle ladite partie perforable et/ou déchirable et/ou décollable est constituée d'un film ou d'une région présentant une épaisseur inférieure par rapport à l'épaisseur de la paroi de fond de capsule.

9. Capsule selon l'une quelconque des revendications précédentes, dans laquelle la paroi externe (104) est reliée au corps interne (102) de capsule par l'intermédiaire de deux cloisons radiales (116) ou plus.

10. Ensemble d'administration pour une substance nébulisée pour une aérosolthérapie ou pour une irrigation nasale, comprenant une capsule (10 ; 1010) selon l'une quelconque des revendications précédentes, contenant la substance à l'état liquide, et un dispositif d'administration (4 ; 400) comprenant :
- un corps de base (8) doté d'une buse (12) de dispositif appropriée pour être reliée fluidiquement à une source d'air comprimé, la buse (12) de dispositif présentant un trou d'administration (14) de l'air comprimé et étant configurée pour s'intégrer dans la buse (110) de capsule, le corps de base (8) définissant une paroi de fond concave (82) pour collecter des substances à partir de laquelle s'étend la buse (12) de dispositif, la paroi de fond concave (82) étant délimitée en périphérie par un bord de couplage de capsule supérieur (84) approprié pour obtenir un couplage de forme et/ou de force avec le bord inférieur (104a) de la paroi latérale externe (104) de la capsule et étant doté de moyens d'ouverture (86) de chambre appropriés pour obtenir au moins une ouverture dans la partie perforable et/ou déchirable et/ou décollable en raison d'un mouvement relatif entre la capsule et le corps de base ;
- un corps d'administration supérieur (30 ; 300) présentant une paroi latérale (32 ; 302) formant un bord inférieur (32a ; 302a) pour relier la capsule, approprié pour obtenir un couplage de forme et/ou de force avec le bord supérieur (104b) de la paroi latérale externe de la capsule, le corps d'administration supérieur étant doté d'une ouverture d'administration (34 ; 304) de la substance nébulisée appropriée pour acheminer vers l'extérieur la substance nébulisée sortant de la capsule.

11. Ensemble selon la revendication précédente, dans lequel le corps d'administration supérieur (30) forme une paroi de fond (38), et un siphon interne (36) s'étendant depuis ladite paroi de fond (38) et qui présente une ouverture d'entrée (36a) agencés de manière à recevoir la substance sortant de la buse (112) de capsule et une ouverture de sortie (36b) opposée à l'ouverture d'entrée, et dans lequel, dans une configuration d'assemblage de l'ensemble, la paroi latérale (32), la paroi de fond (38) et le siphon interne (36) délimitent une chambre de collecte de mucus (50) séparée fluidiquement de la capsule sous-jacente, la paroi de fond (38) et l'ouverture d'entrée (36a) du siphon interne (36) étant espacées de la capsule (10) pour permettre un reflux de la substance nébulisée du siphon interne (36) à la paroi concave (82) pour collecter la substance à travers le canal de récupération de substance (114).

12. Ensemble selon la revendication précédente, dans lequel un capuchon d'administration (60) est monté sur le siphon interne (36), le capuchon se terminant par une pointe d'administration (62) appropriée pour être insérée dans une narine de l'utilisateur, le capuchon d'administration (60) présentant une paroi latérale (64) placée autour d'une partie haute du siphon interne (36), des canaux d'évacuation de mucus (66) étant obtenus entre ladite paroi latérale et ladite partie haute dans la chambre de collecte de mucus (50).

13. Ensemble selon la revendication 10, dans lequel la capsule (1010) est dotée de moyens de nébulisation (40) et le corps d'administration supérieur (300) est doté d'une partie tubulaire (306) ouverte au-dessus en direction de l'extérieur et au-dessous vers les moyens de nébulisation (40), lorsque le corps d'administration supérieur (300) est relié à la capsule, de manière à permettre une aspiration d'air depuis l'extérieur par un effet Venturi, l'ouverture d'administration (304) étant en communication avec une chambre de nébulisation (308) obtenue autour de la partie tubulaire (306).

14. Ensemble selon la revendication précédente, dans lequel, dans une configuration d'assemblage de l'ensemble, la chambre de nébulisation (308) est en communication fluidique avec le canal de récupération de substance (114) de la capsule (10).
